# FASCICULE DE BREVET EUROPEEN

(11) **EP 0 761 168 B1**
(45) Date de publication et mention de la délivrance du brevet: **23.04.2003**
(21) Numéro de dépôt: 96401774.3
(22) Date de dépôt: 13.08.1996
(51) Int. Cl.: A61B 7/02

(54) **Stéthoscope biauriculaire à écoute environnementale**
Stethoskop mit zwei Ohrendstücken, womit die Geräusche der Umgebung erfasst werden können
Binaural stethoscope, capable of hearing the outside surroundings

(30) Priorité: 29.08.1995 FR 9510156
(43) Date de publication de la demande: 12.03.1997
(73) Titulaire: Boussignac, Georges, 92160 Antony (FR)
(72) Inventeur: Boussignac, Georges, 92160 Antony (FR)
(74) Mandataire: Bonnetat, Christian

(56) Documents cités:
- WO-A-81/00353
- AU-A- 3 000 377
- FR-A- 2 621 806
- GB-A- 2 051 584
- US-A- 2 271 467
- US-A- 3 314 499

## Description

La présente invention concerne un stéthoscope biauriculaire à écoute environnementale.

On sait que les stéthoscopes biauriculaires comportent deux embouts auriculaires destinés à venir respectivement en appui contre l'entrée des conduits auriculaires d'un praticien et reliés à une sonde de captage de sons, appliquée par ledit praticien à certains endroits du corps d'un patient. A cet effet, le praticien tient la sonde dans une de ses mains et l'applique contre le corps du patient pendant l'auscultation. Les deux embouts auriculaires sont reliés à la sonde de captage de sons par une voie de transmission des sons captés qui, généralement, comporte deux branches tubulaires rigides portant, d'un côté, les embouts auriculaires et réunies, de l'autre côté, à ladite sonde par un système de tube souple en T, faisant partie de ladite voie de transmission.

Lorsque le praticien a introduit lesdits embouts auriculaires dans ses oreilles et qu'il a appliqué la sonde contre le corps du patient, il n'entend pas, ou tout au moins non distinctement, les bruits de l'environnement, seuls les sons provenant du corps du patient lui étant transmis par le stéthoscope.

Dans ces conditions, lorsque pendant une auscultation le patient parle au praticien, ce dernier ne peut comprendre ce qui lui est dit. Or, l'expérience a montré que pendant une telle auscultation les patients se livraient plus intimement à leur praticien et fournissaient à celui-ci des indications précieuses quant à l'établissement d'un diagnostic. Bien entendu, le praticien est alors amené à écarter au moins l'un des embouts auriculaires de son oreille et à demander au patient de répéter ce qui lui a été précédemment dit. Le charme est alors rompu et le patient ne répète pas exactement les confidences précédentes ou même reste muet.

Par ailleurs, au cours d'une auscultation, des bruits environnementaux extérieurs peuvent venir parasiter les sons écoutés par le praticien à travers le stéthoscope et le praticien peut être amené à vouloir connaître exactement la nature des sons parasites.

On remarquera que le document FR-A-2 621 806 décrit un stéthoscope pour praticiens malentendants devant porter des prothèses auditives. Ce stéthoscope connu est "aisément commutable grâce au maniement d'un interrupteur, respectivement en un mode d'écoute des bruits d'auscultation ou en un mode d'écoute des conversations, de sorte que, entre les phases d'auscultation, l'écoute et la conversation avec le patient puissent se faire de façon commode sans avoir pour le praticien à enlever ou remettre ses prothèses".

La présente invention a pour objet un stéthoscope usuel, perfectionné pour permettre l'écoute environnementale sans que le praticien ait à modifier la position de sa main prenant appui sur le patient, ni celle de son autre main appliquant la sonde contre ce dernier.

A cette fin, selon l'invention, le stéthoscope comportant deux embouts auriculaires reliés à une sonde de captage de sons par une voie de transmission des sons captés, ladite sonde étant tenue par une main d'un praticien pendant l'auscultation d'un patient et comportant deux pavillons opposés, susceptibles d'être alternativement mis en communication avec une chambre commune débouchant dans ladite voie de transmission, est caractérisé en ce que ladite chambre commune comporte un orifice qui est prolongé vers l'extérieur de ladite sonde par un tube rigide saillant, solidaire de celle-ci, et en ce que l'extrémité dudit tube rigide, opposée audit orifice, est obturable de façon, en position ouverte, à mettre ladite voie de transmission en communication sonore avec l'environnement, l'obturation et l'ouverture de ladite extrémité étant commandées par ladite main tenant ladite sonde.

Ainsi, lorsque pendant une auscultation, le praticien a besoin d'écouter des bruits ou des sons de l'environnement, il peut le faire sans modifier essentiellement son attitude et sa position vis-à-vis du patient, simplement en bougeant l'un de ses doigts de la main tenant la sonde. En effet, pendant l'auscultation proprement dite, c'est-à-dire pendant l'écoute des sons en provenance du patient, le praticien obture avec sa main tenant la sonde l'extrémité dudit tube rigide, alors que lorsque le praticien désire entendre des sons ou bruits extérieurs, il lui suffit d'ouvrir ladite extrémité par action de ladite main. A ce moment, la voie de transmission est ouverte vers l'extérieur de sorte que les sons et bruits de l'environnement parviennent aux oreilles du praticien.

Pour permettre un meilleur captage des sons et également une meilleure obturation de la voie d'écoute parallèle environnementale, l'extrémité dudit tube rigide, opposée audit orifice, peut être évasée en trompette.

Lorsqu'une obturation manuelle est envisagée, l'extrémité évasée dudit tube rigide présente un diamètre tel qu'elle peut être obturée à l'aide de la pulpe d'un seul doigt de ladite main.

Bien entendu, en variante, l'extrémité du tube rigide, opposée audit orifice, peut être obturable à l'aide d'un obturateur mobile commandable par la main du praticien tenant la sonde.

Dans un mode de réalisation particulier, il est avantageux que ledit tube rigide saillant soit opposé, par rapport à ladite chambre commune, à ladite voie de transmission.

Les figures du dessin annexé feront bien comprendre comment l'invention peut être réalisée. Sur ces figures, des références identiques désignent des éléments semblables.

La figure 1 montre un stéthoscope connu.

La figure 2 montre, en coupe axiale, la sonde d'un stéthoscope perfectionné selon la présente invention.

La figure 3 est une vue de face de la sonde de la figure 2.

La figure 4 illustre l'obturation manuelle du tube évasé prévu, conformément à l'invention, sur la sonde des figures 2 et 3.

La figure 5 illustre, en variante, un obturateur prévu pour la commande du passage des sons à travers le tube rigide de la sonde des figures 2 et 3.

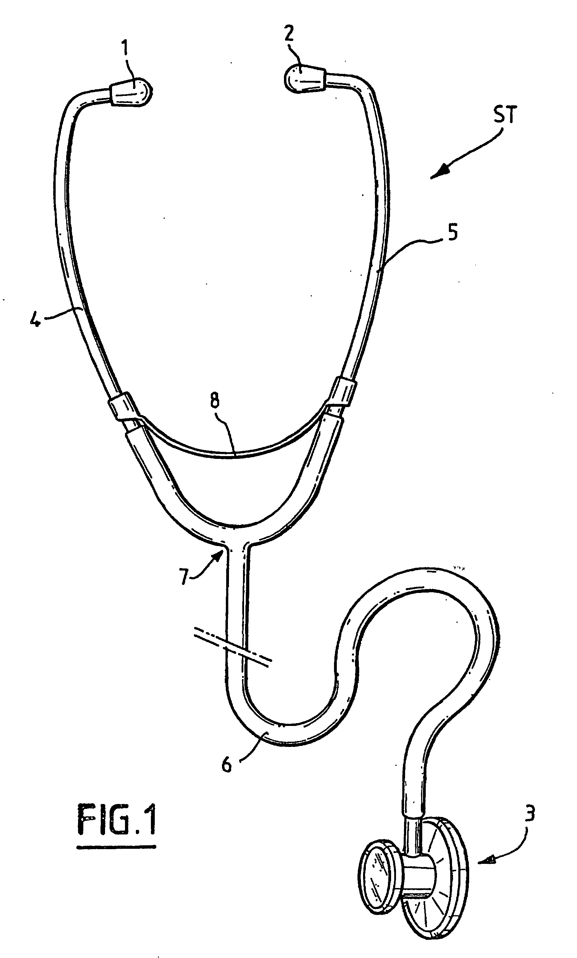

Le stéthoscope connu ST montré schématiquement sur la figure 1 comporte deux embouts auriculaires 1 et 2 reliés à une sonde de captage des sons 3 par une voie de transmission des sons captés comprenant deux branches tubulaires rigides 4 et 5 reliées à un tube souple 6 par un embranchement 7 en forme de T.

Une lame de ressort 8 maintient ensemble les branches rigides 4 et 5 en les rapprochant et la sonde 3 est emmanchée à l'extrémité du tube 6 opposée à l'embranchement 7 en forme de T. Ainsi, en utilisation usuelle du stéthoscope ST, le praticien écarte les branches rigides 4 et 5 du stéthoscope, à l'encontre de l'action de la lame 8 et introduit les embouts auriculaires 1 et 2 respectivement dans ses oreilles, dans lesquelles ils sont maintenus par l'action élastique de la lame 8. Par l'une de ses mains, généralement la main gauche, le praticien prend appui sur le patient et applique avec son autre main, généralement la main droite, la sonde 3 contre une partie du corps dudit patient.

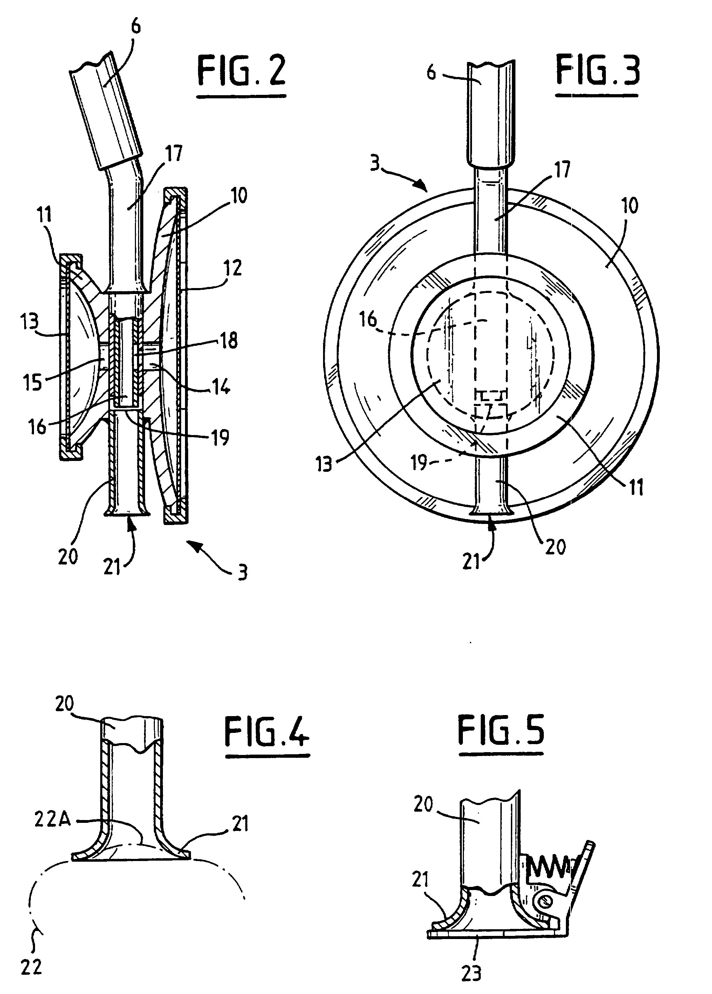

Comme on peut le voir sur les figures 2 et 3, la sonde 3 comporte, de façon usuelle, deux pavillons 10 et 11, de tailles différentes, associés chacun à une membrane vibrante 12 ou 13, destinée à être appliquée contre la peau du patient. Par des ouvertures 14 ou 15, lesdits pavillons peuvent alternativement communiquer avec une chambre commune 16 reliée au tube souple 6 par un embout coudé rigide 17. Cet embout 17 peut tourner à l'intérieur de la sonde 3 de façon à mettre un orifice 18 en regard de l'un ou l'autre des conduits 14 ou 15 et donc relier l'un ou l'autre desdits pavillons 10 et 11 avec les embouts auriculaires 1 et 2.

La sonde 3 telle que décrite ci-dessus est connue.

Conformément à la présente invention, une telle sonde connue est perfectionnée en ce que, dans la paroi de la chambre commune 16, est percé un orifice 19, diamétralement opposé au tube coudé 17. De plus, l'orifice 19 est prolongé vers l'extérieur de ladite sonde par un tube rigide 20 qui en est solidaire, qui est disposé entre lesdits pavillons et qui est évasé à son extrémité 21 opposée à l'orifice 19.

On comprendra aisément que pendant l'auscultation, le praticien peut ouvrir ou fermer l'extrémité évasée 21 du tube 20 à l'aide d'un de ses doigts (par exemple l'index de sa main appliquant la sonde contre le patient) de façon à permettre ou à empêcher la transmission des bruits et des sons extérieurs jusqu'à ses oreilles.

Lorsque le praticien désire n'écouter que les sons provenant du corps du patient, il obture ladite extrémité 21, par exemple au moyen de la pulpe de l'index de sa main tenant la sonde 3 appliquée contre le patient.

En revanche, lorsque le praticien veut écouter des bruits ou des sons extérieurs au patient, il dégage ladite extrémité 21, de sorte que ces bruits et sons extérieurs lui parviennent à travers le tube 6, l'embranchement 7, les branches 4 et 5 et les embouts auriculaires 1 et 2, sans qu'il ait à changer son attitude par rapport au patient ni à écarter l'une ou l'autre ou les deux branches du stéthoscope de ses oreilles.

On remarquera de plus, comme cela est illustré par la figure 4, que si le praticien presse plus ou moins la pulpe d'un de ses doigts contre l'ouverture évasée 21 du tube 20, il fait pénétrer plus ou moins de cette pulpe à l'intérieur dudit tube, modifiant ainsi quelque peu la longueur de la voie de transmission et donc la qualité de la transmission des sons provenant du patient. On peut voir en effet que si la pulpe 22 d'un doigt est légèrement appliquée contre l'ouverture évasée 21, cette pulpe vient affleurer ladite ouverture. En revanche, si la pulpe 22 est fortement appliquée contre l'ouverture évasée 21, une partie de ladite pulpe référencée 22A sur la figure 4 pénètre à l'intérieur du tube rigide 20.

On notera également que, si l'embout rotatif 17 est amené dans une position pour laquelle ledit orifice 18 n'est en regard ni du conduit 14, ni du conduit 15, le praticien élimine complètement la transmission des sons provenant du corps du patient et est apte à n'entendre que la voix de ce dernier et les sons extérieurs.

En variante à l'obturation manuelle prévue sur les figures 2 à 4, on peut prévoir un obturateur 23, par exemple du type à ressort prévu sur certains instruments de musique à vent, pour obturer l'extrémité évasée 21 (voir la figure 5).

## Revendications

1. Stéthoscope comportant deux embouts auriculaires (1, 2) reliés à une sonde (3) de captage de sons par une voie de transmission des sons captés (4 à 7), ladite sonde (.3) étant tenue par une main d'un praticien pendant l'auscultation d'un patient et comportant deux pavillons opposés (10, 11), susceptibles d'être alternativement mis en communication avec une chambre commune (16) débouchant dans ladite voie de transmission,
**caractérisé en ce que** ladite chambre commune (16) comporte un orifice (19) qui est prolongé vers l'extérieur de ladite sonde par un tube rigide saillant (20), solidaire de celle-ci, et **en ce que** l'extrémité (21) dudit tube rigide (20), opposée audit orifice (19), est obturable de façon, en position ouverte, à mettre ladite voie de transmission en communication sonore avec l'environnement, l'obturation et l'ouverture de ladite extrémité étant commandées par ladite main tenant ladite sonde.

2. Stéthoscope selon la revendication 1,
**caractérisé en ce que** l'extrémité (21) dudit tube rigide (20), opposée audit orifice (19), est évasée en trompette.

3. Stéthoscope selon l'une des revendications 1 ou 2,
**caractérisé en ce que** l'extrémité évasée (21) dudit tube rigide (20) présente un diamètre tel qu'elle peut être obturée à l'aide de la pulpe (22) d'un seul doigt de ladite main.

4. Stéthoscope selon l'une des revendications 1 ou 2,
**caractérisé en ce que** l'extrémité (21) dudit tube rigide (20), opposée audit orifice (19), est obturable à l'aide d'un obturateur mobile (23).

5. Stéthoscope selon l'une quelconque des revendications 1 à 4,
**caractérisé en ce que** ledit tube rigide saillant (20) est opposé, par rapport à ladite chambre commune (16), à ladite voie de transmission (17, 4 à 7).

## Claims

1. Stethoscope comprising two earpieces (1, 2) which are connected to an endpiece (3) for capturing sounds via a route for transmission (4 to 7) of the captured sounds, said endpiece (3) being held in one hand by a practitioner during auscultation of a patient and comprising two opposite bells (10, 11) which are capable of being brought alternately into communication with a common chamber (16) opening into said transmission route, **characterized in that** the said common chamber (16) comprises an orifice (19) which is continued outwards from said endpiece via a rigid projecting tube (20) integral therewith, and **in that** the extremity (21) of said rigid tube (20), which is opposite said orifice (19), can be closed so that, in the open position, it is capable of bringing said transmission route into sound communication with the surroundings, the closure and opening of said extremity being controlled by said hand holding said endpiece.

2. Stethoscope according to claim 1, wherein the extremity (21) of the said rigid tube (20) opposite said orifice (19) is flared in a trumpet shape.

3. Stethoscope according to either of claims 1 and 2, **characterized in that** the flared extremity (21) of said rigid tube (20) has a diameter which is such that it can be closed with the aid of the pulp (22) of a single finger of the said hand.

4. Stethoscope according to either of Claims 1 and 2, **characterized in that** the extremity (21) of said rigid tube (20) opposite said orifice (19) can be closed with the aid of a movable closing piece (23).

5. Stethoscope according to claim 4, **characterized in that** said rigid projecting tube (20) is on the opposite side of said common chamber (16) from said transmission route (17, 4 to 7).

## Patentansprüche

1. Stethoskop, das zwei Ohrenendstücke (1, 2) umfasst, die über einen Kanal zur Übertragung der erfassten Geräusche (4 bis 7) mit einer Geräuscherfassungssonde (3) verbunden sind, wobei die genannte Sonde (3) während des Abhörens eines Patienten von einer Hand des praktischen Arztes gehalten wird und zwei gegenüberliegende Hörmuscheln (10, 11) umfasst, die wechselweise mit einer gemeinsamen Kammer (16), die in den genannten Übertragungskanal mündet, verbunden werden können,
**dadurch gekennzeichnet, dass** die genannte gemeinsame Kammer (16) eine Öffnung (19) umfasst, die zur Außenseite der genannten Sonde hin durch ein starres vorstehendes Rohr (20) verlängert wird, das fest mit dieser Sonde verbunden ist, und dadurch, dass das Ende (21) des genannten starren Rohrs (29), das sich gegenüber der genannten Öffnung (19) befindet, so verschließbar ist, dass es in geöffneter Stellung den genannten Übertragungskanal in Geräuschverbindung mit der Umgebung bringt, wobei das Verschließen und das Öffnen des genannten Endes durch die genannte Hand, die die genannte Sonde hält. gesteuert werden.

2. Stethoskop gemäß Anspruch 1,
**dadurch gekennzeichnet, dass** das Ende (21) des genannten starren Rohrs (20), das sich gegenüber der genannten Öffnung (19) befindet, trompetenförmig aufgeweitet ist.

3. Stethoskop gernäß Anspruch 1 oder 2,
**dadurch gekennzeichnet, dass** das aufgeweitete Ende (21) des genannten starren Rohrs (20) einen solchen Durchmesser besitzt, dass es mit der weichen Masse (22) eines einzigen Fingers der genannten Hand verschlossen werden kann.

4. Stethoskop gemäß Anspruch 1 oder 2,
**dadurch gekennzeichnet, dass** das Ende (21) des genannten starren Rohrs (20), das sich gegenüber der genannten Öffnung (19) befindet, mittels eines beweglichen Verschlusses (23) verschließbar ist.

5. Stethoskop gemäß einem beliebigen der Ansprüche 1 bis 4,
**dadurch gekennzeichnet, dass** das genannte vorstehende starre Rohr (20) in Bezug auf die genannte gemeinsame Kammer (16) gegenüber dem genannten Übertragungskanal (17, 4 bis 7) liegt.
